# EUROPEAN PATENT APPLICATION

(11) **EP 4 261 204 A1**
(43) Date of publication of application: **18.10.2023**
(21) Application number: 22168206.5
(22) Date of filing: 13.04.2022
(51) Int. Cl.: C07C 273/00, F28D 3/02, F28D 3/04

(54) **A FALLING FILM HEAT EXCHANGER**

(71) Applicant: Yara International ASA, 0277 Oslo (NO)
(72) Inventor: Porro, Lino Giovanni, 1040 Etterbeek (BE)
(74) Representative: De Clercq & Partners

(57) **Abstract**

The present disclosure provides a heat exchanging section for a falling film heat exchanger comprising a cylindrical body comprising a fluid inlet for a heating or cooling fluid, and a fluid outlet for a heating or cooling fluid, the cylindrical body comprising an open top end and a bottom plate comprising a plurality of openings; a plurality of vertical tubes located within the cylindrical body, the tubes being cylindrical with a constant diameter and parallel with each other; a plurality of liquid dividers, wherein a liquid divider is located on top of each vertical tube; the cylindrical body comprises two compartments hermetically separated from each other, a top compartment and a bottom compartment, by a first disk comprising a plurality of openings; the top compartment of the cylindrical body is configured to hold a liquid composition, and the bottom compartment is configured to receive a heating or cooling fluid; each vertical tube is received through an opening of the first disk, and through an opening of the bottom plate; wherein each liquid divider has an open top end; each liquid divider comprises a cylindrical body, wherein the cylindrical body comprises one or more openings.

## Description

### Field of the invention

The present disclosure is related to chemical engineering, in particular to the field of heat exchangers.

### Background of the invention

In chemical plants, a heat exchanger is a device that allows to heat up or cool down a fluid, i.e. a liquid or a gas, without direct contact between the fluid and the cooling or heating medium.

There are different designs of such devices, and one of them is the falling film shell-and-tube heat exchanger, hereafter named falling film heat exchanger.

A shell-and-tube heat exchanger is a device comprising three sections: a distributing section, wherein the fluid to be cooled or heated is introduced into the device and separated in sub-streams, a heat exchanging section, which comprises a body and a plurality of tubes, wherein the plurality of tubes directs the fluid from the distributing section to a collecting section wherein the cooled or heated fluid is recovered. The heat exchanging section comprises two spaces: the shell space is the continuous space between the body and the plurality of vertical tubes, wherein the heating or cooling medium is circulated, and the tube space, which is the discontinuous space formed by the collection of the inside of each tube. Typically, the tubes are evenly distributed across the cross section of the shell in a shell-and-tube heat exchanger.

A falling film exchanger is a particular type of shell-and-tube heat exchanger, wherein the exchanger is a vertical top-to-bottom exchanger. The liquid to be heated up or cooled down is directed to the distributing section, which sits at the top of the heat exchanging section. The distributing section comprises a plurality of ferrules or liquid dividers, wherein a liquid divider sits on the top end of each tube of the heat exchanging section. The liquid dividers are designed such that the liquid in the distribution section is directed in the plurality of tubes and creates a film coating the internal circumference of the tube, and falls towards the collection section by gravity. The liquid enters the heat exchanging section in the tubes and exchanges heat with the sides of the tubes that are heated up or cooled by the heating or cooling medium circulating in the shell space of the heat exchanging section. If the heat exchanger is designed to heat up the fluid, some volatile compounds comprised in the liquid may evaporate from the liquid. The gases can flow upwards in the central section of the tubes, and reach the distributing section, wherein a gas outlet allows the gases to exit the exchanger.

A falling film exchanger can be used with different liquids in different industries. One possible application in the fertilizer industry, in particular in the field of urea production, is a carbamate decomposer. A urea-producing plant comprises one or more streams of an aqueous solution comprising ammonium carbamate (NH₄(H₂NCO₂)), urea, and optionally free ammonia. Ammonium carbamate is an intermediate product in the synthesis of urea, and needs to be removed from urea-containing streams, to obtain a pure urea solution. When heated up, ammonium carbamate decomposes into ammonia and carbon dioxide, which are gases at temperatures and pressures used in urea-producing plants. A falling film exchanger can thus be used to remove all of or part of the ammonium carbamate comprised in an aqueous solution comprising urea. The falling film exchanger uses a heating medium, such as steam or saturated steam, in its heat exchanging section to heat up the aqueous solution comprising urea and ammonium carbamate inside the plurality of tubes. The ammonium carbamate breaks down into ammonia and carbon dioxide, which escape the tube via its central section and leave the heat exchanger via a gas outlet. The ammonia and carbon dioxide can be later directed to the synthesis section of the plant to produce more urea.

It may be desirable to increase the exchange surface of the heat exchanging section of a falling film exchanger, which can be done by increasing the number of tubes or increasing the length of the tubes. This increase in surface may allow the exchanger to remove more carbamate from an aqueous solution, or process a larger flow of aqueous solution, or allow the exchanger to operate with a lower steam pressure and/or temperature.

EP1469269A1 (Casale, 2004), DE899795C (Hoechst Ag, 1953) and US3016067 (Henry Vogt Machine Company, 1962) disclose a falling film exchanger, wherein a second heat exchanging section can be connected to the existing heat exchanging section to increase the length of the heat exchanger. The three documents disclose that the second heat exchanging section comprises liquid dividers with a closed top. However, this is not desirable as this design causes the flow of gases escaping the tubes to cross with the liquid flow, which causes disturbance in the flow of the liquid.

There is thus a need to design a way to increase the length of a falling film exchanger such that the liquid and gas flows in the tubes do not cross each other.

### Summary of the invention

The present inventor has designed a new type of liquid divider that may be comprised in a heat exchanging section to be attached to an existing falling film heat exchanger, such that the flows of liquid and gas inside a tube do not cross each other.

In a first aspect, the present disclosure provides a heat exchanging section for a vertical falling film heat exchanger comprising:
- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid;
- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other; and
- a plurality of liquid dividers, wherein a liquid divider is located on top of each vertical tube of the plurality of vertical tubes; wherein:
- each liquid divider has an open top end;
- each liquid divider comprises a cylindrical body, and
- the cylindrical body of each liquid divider comprises one or more openings.

In another aspect, the present disclosure provides a falling film heat exchanger comprising a heat exchanging section according to the present disclosure.

In another aspect, the present disclosure provides a method for modifying a conventional falling film heat exchanger comprising a distribution section, a first heat exchanging section comprising a plurality of vertical tubes, and a collection section, comprising the steps of: a) providing a second heat exchanging section according to the present disclosure having the same number of vertical tubes being arranged in the same geometry as the plurality of vertical tubes of the first heat exchanging section; b) disconnecting the collection section from the first heat exchanging section; c) connecting the second heat exchanging section according to the present disclosure to the first heat exchanging section, such that each tube of the first heat exchanging section is directly above a liquid divider of the second heat exchanging section; and d) connecting the collection section to the second heat exchanging section with the collection section.

In another aspect, the present disclosure provides a method for heating up or cooling down a liquid composition, comprising the steps of: a) providing a falling film heat exchanger according to the present disclosure; b) providing a heating fluid or a cooling fluid to the inlets of the heat exchanging sections of the falling film heat exchanger; c) providing the liquid composition to the inlet of the distribution section of the falling film heat exchanger, thereby heating or cooling the liquid composition in the heat exchanging section of the falling film heat exchanger; d) collecting the heated or cooled liquid composition from the collection section of the falling film heat exchanger.

In another aspect, the present disclosure provides a method for removing ammonium carbamate from an aqueous composition, comprising the steps of: a) providing a falling film heat exchanger according to the present disclosure; b) providing a heating fluid to the inlets of the heat exchanging sections of the falling film heat exchanger; c) providing the aqueous composition comprising ammonium carbamate to the inlet of the distribution section of the falling film heat exchanger, thereby heating the aqueous composition comprising ammonium carbamate in the heat exchanging section of the falling film heat exchanger; d) collecting an aqueous composition depleted of ammonium carbamate from the collection section of the falling film heat exchanger.

### Brief description of the figures

The following description of the figures of a specific embodiment of a system according to the present disclosure is only given by way of example and is not intended to limit the present explanation, its application or use. In the figure, identical reference numerals refer to the same or similar parts and features.
Figure 1 represents a conventional falling film heat exchanger according to the prior art.
Figure 2 represents an embodiment of a heat exchanging section according to the present disclosure.
Figure 3 is detailed representation of the top of a tube of a heat exchanging according to the present disclosure.
Figure 4 represents an embodiment of a liquid divider that can be used in a heat exchanging section according to the present disclosure.
Figure 5 shows a liquid divider according to figure 4 on top of a tube in a heat exchanging section according to the present disclosure.
Figure 6 represents an embodiment of a falling film heat exchanger according to the present disclosure.
Figure 7 is an enlarged view of the junction between the two heat exchanging sections of a falling film heat exchanger according to the present disclosure.
Figure 8 is an embodiment of a second disk that may be comprised in a heat exchanging section according to the present disclosure.
Figure 9 shows another embodiment of a liquid divider according to the present disclosure on top of a tube in a heat exchanging section according to the present disclosure.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a section" refers to one or more than one section.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a heat exchanging section for a vertical falling film heat exchanger comprising:
- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid;
- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other; and
- a plurality of liquid dividers, wherein a liquid divider is located on top of each vertical tube of the plurality of vertical tubes; wherein:
- each liquid divider has an open top end;
- each liquid divider comprises a cylindrical body, and
- the cylindrical body of each liquid divider comprises one or more openings.

The heat exchanging section according to the present disclosure comprises many features in common with a conventional heat exchanging section of a conventional shell-and-tube heat exchanger:
- a cylindrical body comprising a fluid inlet for a heating or cooling fluid, and a fluid outlet for a heating or cooling fluid, the cylindrical body comprising an open top end and a bottom plate comprising a plurality of openings;
- a plurality of vertical tubes located within the cylindrical body, the tubes being cylindrical with a constant diameter and parallel with each other;
- the cylindrical body comprises two compartments hermetically separated from each other, a top compartment and a bottom compartment, by a first disk comprising a plurality of openings;
- the top compartment of the cylindrical body is configured to hold a liquid composition, and the bottom compartment is configured to receive a heating or cooling fluid;
- each vertical tube is received through an opening of the first disk, and through an opening of the bottom plate.

The plurality of vertical tubes is evenly distributed across a cross-section of the cylindrical body. The bottom plate and the first disk comprise a number of openings equal to the number of tubes comprised in the heat exchanging section. The first disk may be referred to as the tubesheet.

The bottom compartment comprises two spaces: the shell space is the continuous space between the body and the plurality of vertical tubes, wherein the heating or cooling fluid is circulated, and the tube space, which is the discontinuous space formed by the collection of the inside of each vertical tube.

The heat exchanging section according to the present disclosure further comprises a plurality of liquid dividers located at the top of each vertical tube. These liquid dividers ensure that, in operation, the liquid to be cooled or heated can reach inside the tubes of the heat exchanger, and form a film coating the inside of the vertical tubes; and that the gases evaporating from the liquid can freely flow to the top of a tube and through a liquid divider without crossing the path of the falling liquid. So a new type of liquid divider was designed, wherein:
- each liquid divider has an open top end;
- each liquid divider comprises a cylindrical body comprising one or more openings.

The open top end allows the gases evaporating from the liquid to flow through the liquid divider without crossing the path of the falling liquid. The one or more openings allow the liquid to flow inside the liquid divider and the tube. The cylindrical body of the liquid divider may have the same diameter or a smaller diameter than the vertical tubes.

In one embodiment, the cylindrical body of the liquid divider has a diameter equal to from 50% to 100% of the diameter than the vertical tubes.

In one embodiment, each liquid divider comprises a cylindrical body with a diameter equal or smaller than the diameter of the vertical tubes, and a top end being a truncated cone, wherein the diameter at the top of the liquid divider is less than the diameter of the cylindrical body of the liquid divider

In one embodiment, each liquid divider comprises two or more openings, such as for instance four openings, in its cylindrical body. It may be an advantage for the liquid divider to comprise more than one opening, particularly evenly spaced around the circumference of the cylindrical body in the same cross-section of the liquid divider, so that the liquid is flowing evenly into the liquid divider from several openings, and creates a film on the entire inner surface of the liquid divider in a shorter distance. In one embodiment, each liquid divider comprises at least four openings in its cylindrical body.

In one embodiment, each liquid divider comprises 1, 2, 3, 4, 5, 6, 7, 8, 9, or 10 openings.

In one embodiment, the two or more openings are comprised in the same cross-section of the liquid divider. Having the openings comprised in the same cross-section of the liquid divider ensures that the liquid always enters the liquid divider via the two or more openings at the same height.

In one embodiment, a liquid divider comprises four openings, the four openings being comprised in the same cross-section of the liquid divider and are spaced 90 °, thereby forming a cross. It was observed that a symmetrical distribution of the openings on the cross-section of the liquid divider, i.e. such that the four openings form an imaginary cross in that cross-section, allows the film to form faster inside the liquid divider.

In one embodiment, the liquid dividers comprise a second cylindrical section connected to its cylindrical body. The second cylindrical section of a liquid divider may be configured to be inserted into a vertical tube of the heat exchanging section. In that embodiment, the external diameter of the second cylindrical section is at most equal to the internal diameter of the vertical tubes of the heat exchanging section, at least 0.01 mm, at least 0.05 mm, at least 0.1 mm, at least 0.2 mm, at least 0.3 mm, at least 0.4 mm, or at least 0.5 mm smaller than the internal diameter of the vertical tubes of the heat exchanging section.

Alternatively, the second cylindrical section of the liquid divider may be configured to surround the top end of a vertical tube of the heat exchanging section. In that embodiment, the internal diameter of the second cylindrical section is at least equal to the external diameter of the vertical tubes of the heat exchanging section, at least 0.01 mm, at least 0.05 mm, at least 0.1 mm, at least 0.2 mm, at least 0.3 mm, at least 0.4 mm, or at least 0.5 mm greater than the external diameter of the vertical tubes of the heat exchanging section. This section increases the stability of the liquid divider when sitting on top of a tube.

If the second cylindrical section of the liquid divider is configured to surround the top end of a vertical tube of the heat exchanging section, the liquid divider may comprise a gasket, in particular a gasket made of a fluorinated polymer, such as polytetrafluoroethylene, also known as PTFE. The gasket is located between the tube and the second cylindrical section of the liquid divider to increase the stability of the liquid divider.

In one embodiment, the external diameter of the second cylindrical section is from 0.01 to 0.5 mm smaller to the internal diameter of the vertical tubes of the heat exchanging section.

In one embodiment, the internal diameter of the second cylindrical section is from 0.01 to 3.0 mm, from 0.01 to 1.0 mm, from 1.0 to 3.0 mm, or from 0.01 to 0.5 mm greater to the external diameter of the vertical tubes of the heat exchanging section.

In one embodiment, the heat exchanging section further comprises a second disk located above the first disk, the second disk comprising a plurality of first openings for receiving the plurality of liquid dividers, and a plurality of second openings for allowing the liquid composition to pass through the second disk. A second disk comprising two series of openings, wherein one series is configured to receive the plurality of liquid dividers and the other series is configured to let the liquid go through, increases the rigidity of the system by reducing the vibrations of the liquid dividers in operation.

In one embodiment, the series of openings of the second disk, that are configured to receive the liquid dividers, are cylindrical. The series of openings of the second disk, that are configured let a fluid go through, may have any type of design, and are typically evenly distributed across the area of the second disk.

In one embodiment, the series of openings of the second disk, that are configured let a liquid go through, are cylindrical. Openings with a cylindrical shape may be easier to manufacture.

In one embodiment, the diameter of the openings configured to receive the plurality of liquid dividers is larger than the outer diameter of the liquid dividers, such that a liquid composition may pass through the second disk. In one embodiment, the diameter of the openings configured to receive the plurality of liquid dividers is 0.05, 0.1, 0.15, 0.2, 0.3, 0.4, 0.5, 0.6, 0.7, 0.8, 0.9 or 1mm larger than the outer diameter of the liquid dividers. The larger the openings are, the more liquid composition is able to pass through.

In one embodiment, the second openings for allowing the fluid composition to pass through the second disk are cylindrical, in particular cylindrical with a diameter of from 5 to 10 mm.

In one embodiment, the inner diameter of the top end of the liquid dividers is from 10% to 80%, from 15% to 80%, from 20% to 80%, from 10% to 70%, or from 20% to 70% smaller than the inner diameter of the cylindrical body of the liquid dividers. The diameter should be smaller than the diameter of the tubes, so that the liquid film does not fall directly inside the liquid divider. The top end of the liquid divider should be large enough such that the gases evaporating from the tubes can flow freely out of the liquid divider without causing a pressure increase. In one embodiment, the inner diameter of the top end of the liquid dividers is equal to from 10% to 80%, from 15% to 80%, from 20% to 80%, from 10% to 70%, from 20% to 70%, from 30% to 50% of the inner diameter of the cylindrical body of the liquid dividers.

In one embodiment, the one or more openings of the liquid dividers are located from 30 to 100 mm above the first disk. The height of the openings of the liquid dividers determines the amount of liquid than can accumulate in the top compartment of the heat exchanging section.

In another aspect, the present disclosure provides a falling film heat exchanger comprising a heat exchanging section according to the present disclosure. It is possible to build a falling film heat exchanger comprising two heat exchanging sections, the first, or upper, heat exchanging section being directly above the second, or lower, heat exchanging section, with the second or lower heat exchanging section being a heat exchanging section according to the present disclosure. The falling film heat exchanger also comprises a distribution section above the upper heat exchanging section, and a collection chamber below the lower heat exchanging section.

The falling film heat exchanger comprises one inlet for a liquid to be heated up or cooled down in the distribution section, one outlet for a liquid to be heated up or cooled down in the collection section, and two inlets and two outlets for providing a heating or cooling medium to the shell side space of each exchanging section, each exchanging section comprising one inlet and one outlet fluidly connected to their body. The upper and lower heat exchanging section each comprise a plurality of vertical tubes. The two heat exchanging sections comprise the same number of vertical tubes, which are arranged in the same geometry or design, such that each tube of the upper heat exchanging section is directly above a tube of the lower heat exchanging section.

In one embodiment, the collection section of the falling film heat exchanger comprises an inlet for a passivation gas. It may be required to inject a passivation gas in the tubes of a heat exchanger to prevent or reduce corrosion. This is done by directing a stream of gas comprising oxygen gas into the collection chamber of the heat exchanger. The stream of gas travels within the vertical tubes is evacuated from the distribution chamber via a gas outlet. Without wishing to be bound by theory, the oxygen gas reacts with the tubes, which may be made of stainless steel, and create a layer of oxidized material which is resistant to chemical corrosion caused by the liquid composition, in particular if the liquid composition comprises ammonium carbamate. The passivation gas may further comprise any component selected from the group consisting of ammonia, carbon dioxide, water, and nitrogen gas.

In another aspect, the present disclosure provides a method for modifying a conventional falling film heat exchanger comprising a distribution section, a first heat exchanging section comprising a plurality of vertical tubes, and a collection section, comprising the steps of: a) providing a second heat exchanging section according to the present disclosure having the same number of vertical tubes being arranged in the same geometry as the plurality of vertical tubes of the first heat exchanging section; b) disconnecting the collection section from the first heat exchanging section; c) connecting the second heat exchanging section according to the present disclosure to the first heat exchanging section, such that each tube of the first heat exchanging section is directly above a liquid divider of the second heat exchanging section; and d) connecting the collection section to the second heat exchanging section .

A conventional falling film heat exchanger can be modified to have longer heat exchanging tubes, i.e. an increased heat transfer capacity, by introducing a heat exchanging section according to the present disclosure in a conventional heat exchanger.

The conventional falling film heat exchanger comprising a distribution section, a first heat exchanging section, and a collection section, needs to be partially dismantled: the collection section located below the first heat exchanging section needs to be disconnected from the first heat exchanging section. This operation exposes the bottom of the heat exchanging section and the lower end of the vertical tubes. A heat exchanging section according to the present disclosure is provided. The additional heat exchanging section needs to be fully compatible with the first heat exchanging section, i.e. it must have the same number of vertical tubes which are arranged in the same geometry as the existing heat exchanging section. The new heat exchanging section is connected to the first heat exchanging section, becoming the second, or lower, heat exchanging section, such that each tube of the first heat exchanging section is directly above a tube of the second heat exchanging section. This ensures that, in operation, the gases evaporating from the liquid composition in the lower heat exchanging section can freely travel upwards to the distribution section of the heat exchanger without crossing the falling liquid composition.

In one embodiment, the top end of the liquid dividers are comprised within the tubes of the first heat exchanging section. In one embodiment, the top end of the liquid dividers are below the bottom end of the tubes of the first heat exchanging section.

Finally, the collection section that was disconnected from the first heat exchanging section in step b) is connected to the lower heat exchanging section.

In one embodiment, the heat exchanging section provided in step a) has the same external diameter as the first heat exchanging section and/or of the collection chamber of the heat exchanger. If all the sections have the same external diameter, connecting the sections together may be easier and require less work.

In another aspect, the present disclosure provides a method for heating up or cooling down a liquid composition, comprising the steps of: a) providing a falling film heat exchanger according to the present disclosure; b) providing a heating fluid or a cooling fluid to the inlets of the heat exchanging sections of the falling film heat exchanger; c) providing the liquid composition to the inlet of the distribution section of the falling film heat exchanger; d) collecting the heated or cooled liquid composition from the collection section of the falling film heat exchanger.

The falling film heat exchanger according to the present disclosure comprising two heat exchanging sections can be operated in a similar manner as a conventional falling film heat exchanger comprising only one heat exchanging section, with the difference that the shell side space of both heat exchanging sections must be supplied with a heating fluid, such as steam or steam condensate. The two heat exchanging sections may be supplied with the same heating fluid, for example steam at the same temperature and pressure, or a with two different heating fluids.

In one embodiment, both heating fluid inlets of the heat exchanging sections are provided with saturated steam, wherein the first inlet is provided with saturated steam at a first temperature and a first pressure, and the second inlet is provided with saturated steam at a second temperature and a second pressure.

In one embodiment, the falling film heat exchanger is a medium-pressure carbamate decomposer or a low-pressure carbamate decomposer.

In one embodiment, the heating fluid provided in step b) is saturated steam at a pressure of from 6 to 12 bars, or saturated steam at a pressure of from 3 to 5 bars.

In one embodiment, the two heat exchanging sections of the falling film heat exchanger are provided with the same heating fluid, in particular wherein the heating fluid is saturated steam, more in particular wherein the heating fluid is saturated steam at a pressure of from 3 to 12 bar.

The heating fluid is distributed to the shell space of the heat exchanging section and heat up the vertical tubes.

When the equipment is started after a break, it may be an advantage to wait after providing the heating fluid to the heat exchanging sections, such that the vertical tubes are heated up by the heating fluid and reach a desired temperature. If the liquid composition to be heated up is provided too early, the first portion thereof receives less heat when passing through the tubes than designed.

Once the equipment has reached a suitable internal temperature, the liquid composition to be heated up can be provided to the inlet of the distribution section of the heat exchanger. The distribution section ensures that the liquid composition is evenly distributed to the plurality of vertical tubes, and the liquid forms a film inside the tubes and falls due to gravity. While falling, the liquid exchanges heat with the walls of the tubes and heats up. Volatile compounds present in the liquid may evaporate and flow upwards to the distribution section. Chemical reactions may also happen as a consequence of the liquid heating up, such as the decomposition of ammonium carbamate into ammonia and carbon dioxide.

The heated liquid can then be collected from the collection section and be directed to the next processing step of the plant. The liquid composition collected from the collection section may or may not have the same chemical composition as the liquid composition that entered the distribution section.

The heat exchanger may be used as a concentrator, i.e. to evaporate water from an aqueous solution to increase the concentration of the chemical component or components comprised in the aqueous solution.

In another aspect, the present disclosure provides a method for removing ammonium carbamate from an aqueous solution, comprising the steps of: a) providing a falling film heat exchanger according to the present disclosure; b) providing a heating fluid to the inlets of the heat exchanging sections of the falling film heat exchanger; c) providing the aqueous solution comprising ammonium carbamate to the inlet of the distribution section of the falling film heat exchanger; d) collecting an aqueous solution depleted of ammonium carbamate from the collection section of the falling film heat exchanger.

As mentioned above, a falling film heat exchanger may be used to purify and/or concentrate an aqueous solution comprising ammonium carbamate, and optionally urea. When a liquid composition is directed to a falling film heat exchanger, the ammonium carbamate comprised in the solution decomposes back to ammonia and carbon dioxide. Since ammonia and carbon dioxide are gases at the pressure and temperature of the heat exchanger, they evaporate from the solution in the vertical tubes. Water is also evaporated in the heat exchanger. So the aqueous solution recovered from the collection section is an aqueous solution depleted of ammonium carbamate, and optionally comprising urea.

The aqueous solution may still comprise some ammonium carbamate depending on the operating conditions, but the concentration of ammonium carbamate at the bottom of the heat exchanger is less than the concentration in ammonium carbamate at the top of the heat exchanger.

In one embodiment, the aqueous solution provided in step c) comprises from 20 to 30 weight% of ammonia, and 5 to 10 weight% of carbon dioxide. These amounts are typical for an aqueous solution provided to a medium-pressure carbamate decomposer in a urea-producing plant.

In one embodiment, the aqueous solution provided in step c) comprises from 5 to 10 weight% of ammonia, and 1 to 5 weight% of carbon dioxide. These amounts are typical for an aqueous solution provided to a low-pressure carbamate decomposer in a urea-producing plant.

In one embodiment, the aqueous solution provided in step c) comprises urea.

In one embodiment, the falling film heat exchanger provided in step a) is a low-pressure, or a medium-pressure, carbamate decomposer.

In a medium-pressure carbamate decomposer, the aqueous solution comprising urea and ammonium carbamate directed to the decomposer may have a temperature of from 135 to 155 °C, or from 140 to 145 °C. In a medium-pressure carbamate decomposer, the aqueous solution depleted of ammonium carbamate and collected in the collection chamber of the decomposer may have a temperature of from 150 to 170 °C, or from 155 to 165 °C.

In a low-pressure carbamate decomposer, the aqueous solution comprising urea and ammonium carbamate directed to the decomposer may have a temperature of from 110 to 130 °C, or from 115 to 125 °C. In a low-pressure carbamate decomposer, the aqueous solution depleted of ammonium carbamate and collected in the collection chamber of the decomposer may have a temperature of from 130 to 160 °C, or from 135 to 150 °C.

In one embodiment, the aqueous solution collected in step d) comprises from 1 to 3 weight%, or from 1 to 2.5 weight% of ammonia, and from 0 to 1.0 weight%, or from 0.5 to 1.0 weight% of carbon dioxide. These amounts are typical for an aqueous solution collected from a low-pressure carbamate decomposer in a urea-producing plant.

In one embodiment, the aqueous solution collected in step d) comprises from 5 to 10 weight%, or from 5 to 8 weight% of ammonia, and from 1 to 5 weight%, or from 1 to 3 weight% of carbon dioxide. These amounts are typical for an aqueous solution collected from a medium-pressure carbamate decomposer in a urea-producing plant.

In another aspect, the present disclosure provides the use of the falling film exchanger according to the present disclosure for removing ammonium carbamate from an aqueous solution comprising ammonium carbamate, and urea.

In another aspect, the present disclosure provides the use of the falling film exchanger according to the present disclosure for evaporating water from an aqueous solution.

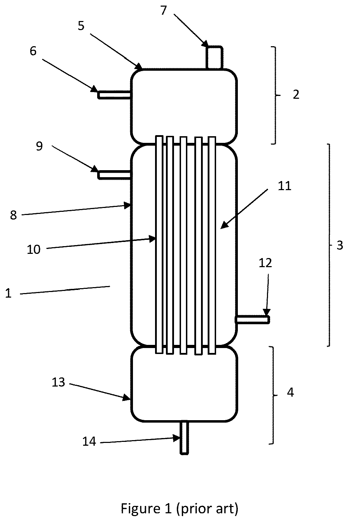

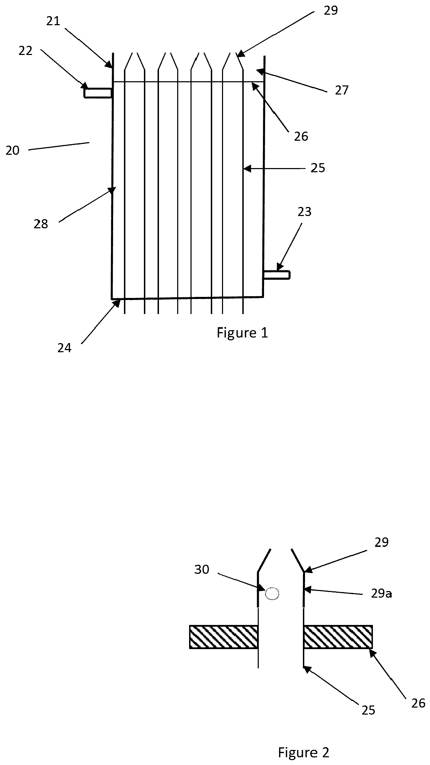

Figure 1 represents a conventional falling film heat exchanger **1.** The heat exchanger **1** comprises a distribution section **2,** a heat exchanging section **3,** and a collection section **4.** The distribution section **2** comprises a container or chamber **5,** which may have any particular geometry, such as hemi-spherical or cylindrical, an inlet **6** for a liquid composition, and an outlet **7** for gases. The heat exchanging section **3** comprises a cylindrical body **8,** an inlet **9** for a heating or cooling fluid, a plurality of vertical tubes **10,** and an outlet **12** for a heating or cooling fluid. The plurality of tubes **10** is evenly distributed across the cross section of the heat exchanging section **2.** Some tubes are omitted from the figure, such that the figure is easier to read and interpret. The tubes **10** fluidly connect the distribution section **2** and the collection section **4.** The body **8** and the tubes **10** create a continuous shell space **11,** hermetically sealed from the distribution and collection section, wherein the heating or cooling fluid can freely move. The collection section **4** comprises a body **13,** and an outlet **14** for a liquid composition.
Figure 2 represents an embodiment of a heat exchanging section according to the present disclosure. The heat exchanging section **20** comprises a body **21,** in particular a cylindrical body, an inlet **22** for a cooling or heating medium, and outlet for a cooling or heating fluid **23,** a bottom plate **24** comprising a plurality of openings for receiving the plurality of tubes **25,** and a disk **26** dividing the body into two compartments hermetically separated from each other, i.e. an upper compartment **27,** and a lower compartment **28.** The heat exchanging section **20** comprises a plurality of tubes **25** distributed evenly across its cross-section. The disk **26** comprises a plurality of openings to receive the tubes **25.** The upper compartment **27** is configured to hold some of the liquid composition before it falls further in the vertical tubes **25.** Each vertical tube **25** comprise a liquid divider **29** on its top end.

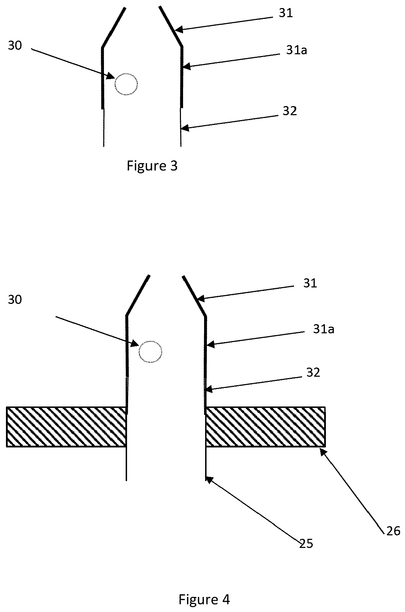

Figure 3 is detailed representation of the top of a tube **25** with a liquid divider **29** of the heat exchanging section **20** shown in figure 2. The vertical tube **25** goes through the disk **26** and a liquid divider **29** is mounted on the top end of the tube **25.** The liquid divider **29** comprises a cylindrical body **29a** which has the same diameter as the tube **25,** and a top end being a truncated cone, wherein the diameter at the top of the liquid divider is smaller than the diameter of its body **29a.** The liquid divider comprises one or more openings **30** in its cylindrical body.
Figure 4 represents an embodiment of a liquid divider **31** that can be used in the heat exchanging section according to the present disclosure. The liquid divider **31** comprises the same features as liquid divider **29** of figure 3: a cylindrical body **31a** comprising a opening **30,** and a top end in the shape of a truncated cone. In addition, it contains a second cylindrical section **32** attached to its body **31a.** The second cylindrical section **32** has an external diameter equal to or smaller than the internal diameter of the tube **25.**
Figure 5 shows a liquid divider **31** according to figure 4 sitting on a tube **25** of a heat exchanging section. The second cylindrical section **32** sits inside the tube **25.** The section **32** increases the stability of the liquid divider **31** in the tube **25.**

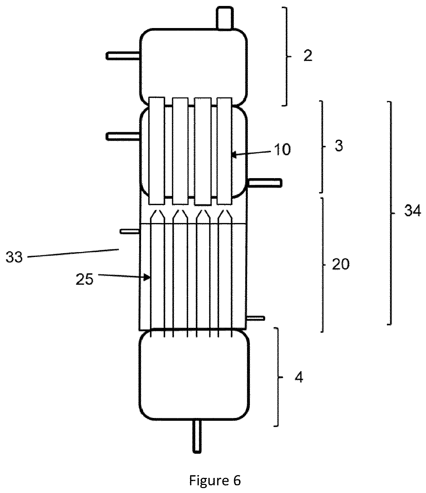

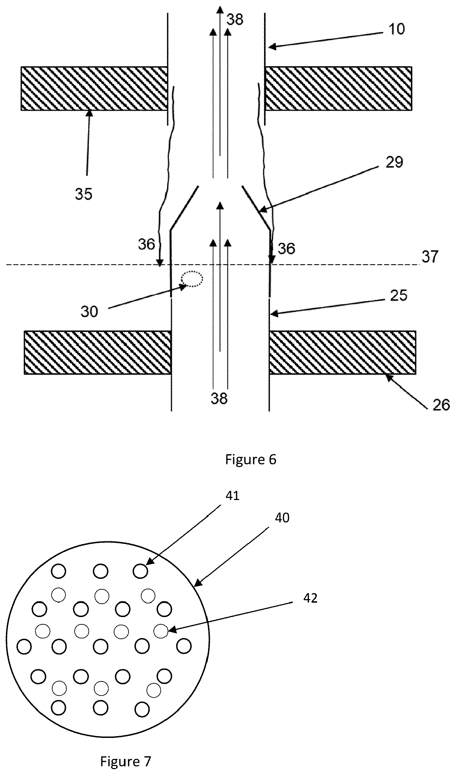

Figure 6 represents an embodiment of a falling film heat exchanger according to the present disclosure. The heat exchanger **33** comprises a distribution section **2,** a collection section **4,** and a heat exchanging module **34** comprising two heat exchanging sections, **3** and **20.** The heat exchanging section **3** is a conventional section, whereas the heat exchanging section **20** is according to the present disclosure. The tubes **10** of the section **3** are aligned with the tubes **25** of the section **20** to ensure a continuous gas flow across the entire length of the heat exchanging module **34.**
Figure 7 is an enlarged view of the area where the two exchanging sections **3** and **21** meet in the heat exchanger **33** in operation. The element **35** represents the bottom plate of the heat exchanging section **3,** and a tube **10** goes though that element **35.** The liquid composition flows downwards and is represented by the two arrows **36.** The dashed line **37** represents the liquid level in the upper compartment of the heat exchanging section in operation. The arrows **38** represent the gases evaporating from the liquid solution and rising to the top of the heat exchanger. It can be seen that the gas flow is confined to the interior of the tubes **10** and **25** at any time, while the liquid flows from the internal sides of tubes **10,** to the outside of tubes **25** and re-enter the tubes **25** via the one or more openings **30.** It can be seen that the gas and liquid flows do not cross each other.

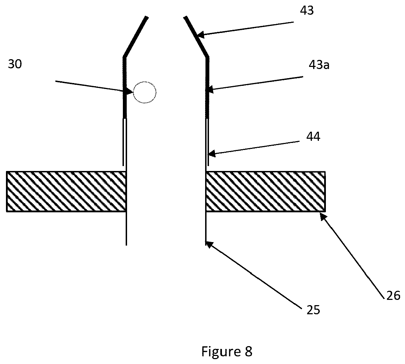

Figure 8 is an embodiment of a second disk **40** that may be comprised in a heat exchanging section according to the present disclosure. The second disk **40** has a diameter that is at most equal to the internal diameter of the cylindrical body of the heat exchanging section. It comprises two series of openings **41** and **42.** The openings **41** are configured to receive the liquid dividers of the heat exchanging section according to the present disclosure. The openings **41** may have a diameter slightly larger, for example from 0.1 to 0.5 mm, than the diameter of the liquid dividers, such that some liquid composition may pass through the openings **41.** The openings **42** are configured to let the liquid composition flowing down the heat exchanger flow though the second disk **42.** The openings **41** and **42** are drawn with a different line thickness, so they are easier to differentiate on a drawing. However, the openings **41** and **42** may have the same or different diameters.
Figure 9 shows another embodiment of a liquid divider **43** according to the present disclosure sitting on a tube **25** of a heat exchanging section. The liquid divider **43** comprises a cylindrical body **43a** comprising an opening **30,** and a top end in the shape of a truncated cone. In addition, it contains a second cylindrical section **44** below its body **43a.** The second cylindrical section **44** has an internal diameter greater than the external diameter of the tubes **25.** The second cylindrical section **44** increases the stability of the liquid divider **43.** A PTFE gasket can be placed between the tube **25** and the second cylindrical section **44.**

## Claims

1. A heat exchanging section (20) for a vertical falling film heat exchanger comprising:
- a cylindrical body (21), comprising a top compartment (27) and a bottom compartment (28) separated from each other by a first disk (26) comprising a plurality of openings, wherein the top compartment (27) has an open top end and the first disk (26) at its bottom end, and wherein the bottom compartment (28) has the first disk (26) as its top end and a bottom plate (24) comprising a plurality of openings as its bottom end, wherein the top compartment (27) of the cylindrical body (21) is configured to hold a liquid composition, and the bottom compartment (28) is configured to receive a heating or cooling fluid, wherein the bottom compartment (28) comprises a fluid inlet (22) for a heating or cooling fluid, and a fluid outlet (23) for a heating or cooling fluid;
- a plurality of vertical tubes (25) located within the cylindrical body (21), the tubes (25) being cylindrical with a constant diameter and parallel with each other, wherein each vertical tube is received through an opening of the first disk (26) and through an opening of the bottom plate (24) and wherein each opening of the first disk (26) and each opening of the bottom plate (24) contains a vertical tube, so that the top compartment (27) and the bottom compartment (28) are hermetically separated from each other; and
- a plurality of liquid dividers (29), wherein a liquid divider (29) is located on top of each vertical tube of the plurality of vertical tubes (25)
**characterized in that:**
- each liquid divider (29) has an open top end;
- each liquid divider (29) comprises a cylindrical body (29a), wherein the cylindrical body (29a) of each liquid divider comprises one or more openings (30).

2. The heat exchanging section according to claim 1, wherein each liquid divider comprises four openings in its cylindrical body, in particular wherein the four openings are comprised in the same cross-section of the liquid divider, in particular wherein the four openings are comprised in the same cross-section of the liquid divider and are spaced 90 °, thereby forming a cross.

3. The heat exchanging section according to claim 1 or 2, further comprising a second disk (40) located between the open top end of the cylindrical body (21) and the first disk (26), the second disk (40) comprising a plurality of first openings (41) for receiving the plurality of liquid dividers (29), and a plurality of second openings (42) for allowing the liquid composition to pass through the second disk (40).

4. The heat exchanging section according to any one of claims 1 to 3, wherein the inner diameter of the top end of the liquid dividers (29) is 20 to 80% smaller than the inner diameter of the cylindrical body (29a) of the liquid dividers (29).

5. The heat exchanging section according to any one of claims 1 to 4, wherein the one or more openings (30) of the liquid dividers (29) are located from 30 to 100 mm above the first disk (26).

6. The heat exchanging section according to any one of claims 1 to 5, wherein the liquid dividers (29, 31, 43) comprise a second cylindrical section (32, 44) connected to its cylindrical body (29a, 31a, 43a).

7. The heat exchanging section according to claim 6, wherein the external diameter of the second cylindrical section (32) of the liquid dividers (31) is from 0.01 to 0.5 mm smaller than the internal diameter of the vertical tubes (25) of the heat exchanging section.

8. The heat exchanging section according to claim 6, wherein the internal diameter of the second cylindrical section (44) of the liquid dividers (43) is from 0.01 to 3.0 mm, or from 0.01 to 0.5 mm greater to the external diameter of the vertical tubes (25) of the heat exchanging section (20).

9. A falling film heat exchanger (33) comprising a distribution section (2), a first heat exchanging section (3), a second heat exchanging section (20) according to any one of claims 1 to 8, and a collection section (4).

10. A method for modifying a conventional falling film heat exchanger comprising a distribution section (2), a first heat exchanging section (3) comprising a plurality of vertical tubes, and a collection section (4), comprising the steps of:
a) providing a second heat exchanging section (20) according to any one of claims 1 to 8 having the same number of vertical tubes (25) being arranged in the same geometry as the plurality of vertical tubes of the first heat exchanging section;
b) disconnecting the collection section (4) from the first heat exchanging section (3);
c) connecting the second heat exchanging section (20) according to any one of claims 1 to 8 to the first heat exchanging section (3), such that each tube of the first heat exchanging section (3) is directly above a liquid divider (29) of the second heat exchanging section (20);
d) connecting the collection section (4) to the second heat exchanging section (20).

11. A method for heating up or cooling down a liquid composition, comprising the steps of:
a) providing a falling film heat exchanger according to claim 9;
b) providing a heating fluid or a cooling fluid to the inlets of the heat exchanging sections of the falling film heat exchanger;
c) providing the liquid composition to the inlet of the distribution section of the falling film heat exchanger;
d) collecting the heated or cooled liquid composition from the collection section of the falling film heat exchanger.

12. A method for removing ammonium carbamate from an aqueous composition, comprising the steps of:
a) providing a falling film heat exchanger according to claim 9;
b) providing a heating fluid to the inlets of the heat exchanging sections of the falling film heat exchanger;
c) providing the aqueous composition comprising ammonium carbamate to the inlet of the distribution section of the falling film heat exchanger;
d) collecting an aqueous composition depleted of ammonium carbamate from the collection section of the falling film heat exchanger.

13. The method according to claim 12, wherein the aqueous composition comprises urea.

14. Use of the falling film exchanger according to claim 9 for removing ammonium carbamate from an aqueous solution comprising ammonium carbamate, and optionally urea.

15. Use of the falling film exchanger according to claim 9 for removing water from an aqueous solution.
